Europäisches Patentamt

European Patent Office

Office européen des brevets

(11) Publication number: **0 167 958**
**B1**

(12) **EUROPEAN PATENT SPECIFICATION**

(45) Date of publication of patent specification: **02.01.91**

(51) Int. Cl.⁵: **A 61 K 9/24**

(21) Application number: **85108146.3**

(22) Date of filing: **01.07.85**

(54) Oral solid pharmaceutical form with sequential action for the administering of drugs with ulcerogenic side effect.

| | |
|---|---|
| (30) Priority: **12.07.84 IT 2187484** | (73) Proprietor: **LABORATORIO ITALIANO BIOCHIMICO FARMACEUTICO LISAPHARMA S.P.A.**<br>**Via Licinio 11, 13, 15**<br>**I-22036 Erba (Como) (IT)** |
| (43) Date of publication of application:<br>**15.01.86 Bulletin 86/03** | |
| (45) Publication of the grant of the patent:<br>**02.01.91 Bulletin 91/01** | (72) Inventor: **Colombo, Paolo**<br>**Via Magenta 12**<br>**Pavia (IT)**<br>Inventor: **Conte, Ubaldo**<br>**Via Strada Persa 7/B**<br>**Pavia (IT)**<br>Inventor: **Zagnoli, Giorgio**<br>**Via Rubini, 7**<br>**Como (IT)** |
| (84) Designated Contracting States:<br>**AT CH DE FR LI** | |
| (56) References cited:<br>**EP-A-0 064 485**<br>**DE-A-3 434 707**<br>**FR-M- 3 715** | |
| | (74) Representative: **Gervasi, Gemma, Dr. et al**<br>**Studio Brevetti e Marchi NOTARBARTOLO & GERVASI 33, Viale Bianca Maria**<br>**I-20122 Milano (IT)** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a new pharmaceutical form with antiinflammatory and analgesic activity, avoiding the ulcerogenic side effect of the antiinflammatory and analgesic active principle.

More particularly, the present invention relates to an oral solid pharmaceutical form with antiinflammatory and analgesic activity, with sequential action, showing an effect of protection of the gastric and duodenal mucosa against the action of the active principles having ulcerogenic side effect.

It is known that during these last years several non-steroidic drugs with antiinflammatory and analgesic action, denominated as FANS (Non Steroidic Antiinflammatory Drugs, NSAD) have been prepared and tested.

It is known too that for all these drugs as a side effect a damaging action on the gastric and duodenal mucosa has been evidenced, which makes unadvisable the prolonged use thereof.

In order to overcome this problem, several modifications and administering forms have been suggested for these drugs, e.g., the formation of salts with alkaline metals, the formation of complexes with Al, Mg or Cu, the preparation of inclusion compounds with B-cyclodextrins and the like.

Notwithstanding the attention devoted to this problem, and the large number of solutions proposed, the problem of the ulcerogenic action of antiinflammatory drugs is to be considered as being still open, and always of great interest in the pharmaceutical field.

A noticeable step forward has been done with the contemporary administering, in the 1:1 ratio, of FANS and sucralfate, which allows an antiinflammatory activity similar to that exerted by the same FANS alone to be obtained, with a considerable reduction in the damaging effects on the gastric and duodenal mucosa (Italian Patent Application Nr. 23205 A.83).

Sucralfate [3,4,5,6-tetra-(polyhydroxyalumino)-α-D-glucopyranosyl sulphate-2,3,4,5-tetra-(polyhydroxyalumino)-β-D-fructofuranoside sulphate] is a product prepared during these last years, and successfully tested in the management of gastric and duodenal ulcer (R. Nagashima et al., Arzneim. Forsch, 1980: 30: 84/8; 1980: 30: 88/91 "Selective Binding of Sucralfate to Ulcer Lesion").

We have now found that if, instead of contemporaneously administering the mixture of the two active principles, the administering in sequence of sucralfate first, and of FANS then is carried out, a more efficacious protection of the mucosa is obtained. The result in practice is such as to allow the same protection level with lower amounts of sucralfate to be obtained. This administering form causes however the drawback that a double adminstering is to be carried out, with a suitable time interval.

Purpose of the present invention is to provide a solid pharmaceutical form which allows the contemporary administering of the two drugs above mentioned, or of other similar drugs, and the release of them in a sequential fashion.

This purpose is achieved by means of the pharmaceutical form sequentially releasing the active principles according to the present invention, which is characterized in that it is constituted by a tablet comprising:

a) a centre core containing an active principle providing an antiinflammatory and analgesic activity with side ulcerogenic effects;

b) a layer coating for said core, containing a second active principle, providing a protective action of the gastric and duodenal mucosa, which is released immediately.

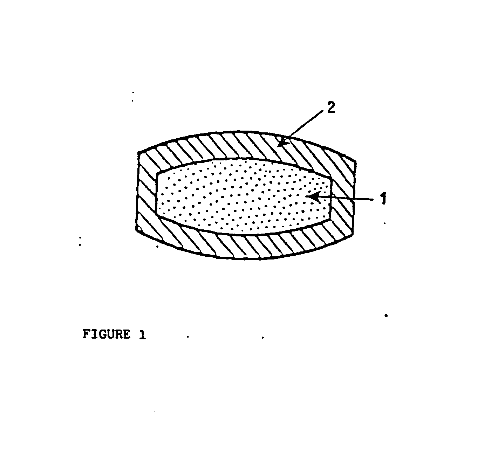

These and other characteristics and advantages of the pharmaceutical form according to the present invention shall be evidenced in greater detail by the following detailed disclosure, and by the related Figure 1, which are reported to the purpose of illustrating and not of limiting the invention itself.

Referring to the numerical indices of Figure 1, the centre core (1) of the pharmaceutical form according to the present invention is prepared by making into a paste the antiinflammatory and analgesic active principle, suitably formulated, with an alcoholic solution of ethylcellulose; the paste is granulated, dried, blended with lubricating and disintegrating substances, and then transformed into tablets.

The coating layer (2) is prepared by mixing sucralfate in suitable formulations, and is applied to the core (1) by means of the double-compression technique, i.e., by compressing two coating layers respectively positioned on and under the core.

The coating layer can contain as mucose protecting active principles, in addition to sucralfate, also mucin, cellulose derivatives, natural or synthetic polymeric materials, alone, or as different combinations with each other.

The coating layer is so formulated, as to release an extremely subdivided dispersion of sucralfate before that the FANS composing the core comes in contact with the gastric and duodenal mucosa.

The action of this pharmaceutical form is hence developed in two sequential steps, whose sequence is evidenced by observations related to tests carried out on animal. The steps of this action are:

J) Disintegration quick and of microgranular type, in the acidic medium of the stomach, of the coating layer, with formation of a wide dispersion of the protective agent composing it. The active principle of the coating has hence the time and the possibility of lining the gastric and intestinal mucosa, protecting it from the subsequent contact with the ulcerogenic drug contained in the core.

B) Slow disintegration of the core in a medium wherein the protective active substance has already lined the mucosa.

In this way, the lesioning action of the drug contained in the core is limited by the action of the protective drug.

Several compositions containing sucralfate: FANS in weight ratios comprised within the range of from 1:4 to 8:1 have been tested on rats by means of the test of pylorus ligature according to the techniuqe Linda J. et al., J. Pharmac., 1978, 30, 244—246 "Inhibitors of Gastric Lesions in the Rat".

The testing has been carried out on Charles River Wistar rats of 230—270 g of weight and in the number of 8 rats per each group. The rats, fasting from 15 hours, have been submitted to etheric anesthesia and then to the ligature of the pylorus. The rats have been treated, immediately after the recovery, by means of gastric probe, with the FANS alone, with the FANS in the sucralfate-FANS form with sequential action according to the present invention, and by means of the administering of sucralfate first, and then, after a 10 minutes interval, of FANS.

The single active principles have been administered as aqueous suspension in sodium-CMC at 0.5% p.o.

Six hours later than the intervention, the rats have been sacrificed and the stomach, after having been withdrawn, has been cut along the line of the greater curvature. The stomach, after having been slightly washed with bidistilled water has been spread out and mounted on a support for the evaluation of induced ulceration. The alterations detected on the gastric mucosa have been quantified on the basis of their type and largeness, with a value ranging from 0 to 1 (ulcerating index, UI), according to the following empirical scale:

0=mucose not damaged (control submitted to surgical handling and to placebo)
0.25=diffused accentuated hyperemia
0.50=diffused erosion
0.75=diffused hemorrhagic ulceration
1=diffused hemorrhagic ulceration with perforation and damaging of the whole gastric mucosa.

The activity of the form of sucralfate and FANS with sequential action according to the invention has been expressed as percentage inhibition of the lesion relatively to that observed in the control group as treated with the ligature of pylorus and administering of FANS only, and compared to that obtained from the contemporary administering of sucralfate and FANS. The $ID_{50}$ (Inhibiting Dosis 50) was computated by the probit method.

In Table 1 the values of $ID_{50}$ of sucralfate for various FANS are reported, in the case of the contemporary administering of FANS and sucralfate, and in the case of the administering in the form with sequential activity according to the present invention.

TABLE 1
Inhibiting Dosis 50 of sucralfate for the ulcerogenic activity of some FANS (mg)

| FANS | Contemporary administering of sucralfate and of FANS | Administering of the sucralfate-FANS form with sequential action |
|---|---|---|
| Sodium indoprofen betainate (200 mg/ kg as indoprofen) | 206 | 85.6 |
| Diclofenac Na (50 mg/kg) | 157 | 96.6 |
| Indomethacin (100 mg/kg) | 204 | 97 |

It results from Table 1 in a clear way that the administering of the form of sucralfate-FANS with sequential action is capable of protecting to a significantly greater extent the gastric mucosa against the lesioning power of FANS.

To illustrative, but not limitative, purpose of the present invention, the following Example is reported, relating to a formulation of the pharmaceutic form with sequential action (the numbers indicate parts by weight):

a) Formulation of the core of sodium indoprofen betainate

| | |
|---|---|
| Sodium indoprofen betainate | 290 |
| Ethylcellulose | 5 |
| Carboxymethyl starch | 12 |
| Magnesium stearate | 3 |

b) Preparation of the core

Indoprofen betainate is made into a paste with an alcoholic solution of ethylcellulose, the paste is granulated and dried. The dried granulate is mixed with the lubricant agent (magnesium stearate) and then with the disintegrating agent (carboxymethyl starch), and is compressed to form tablets of slightly crowned shape, with punches of 9 mm in diameter.

c) Formulation of the coating layer

| | |
|---|---|
| Sucralfate | 100 |
| Crosslinked carboxymethylcellulose | 10 |
| Microcrystalline cellulose | 40 |
| Magnesium carbonate | 10 |
| CL Polyvinylpirrolidone | 5 |
| Magnesium stearate | 2 |

The components of the formulation are mixed in a V-mixer.

d) Application of sucralfate coating on the core of sodium indoprofen betainate

The application of the coating layer on the core is carried out by means of the double-compression technique, by compressing two layers of coating positioned on the core and under it, a coated tablet of suitable diameter being obtained, wherein the outer coating is constituted by sucralfate, and the inner core is constituted by indoprofen betainate (see Figure 1).

**Claims for the Contracting States: CH, DE, FR, LI**

1. Solid pharmaceutical form for administration by oral way, with sequential release of the contained active principles, characterized in that it is constituted by:

a) a central core containing as active principle a non-steroidic antiinflammatory drug which normally causes ulcerogenic side effects;

b) a coating layer for said central core, comprising as active principle sucralfate, which is immediately released in the acidic medium of the stomach,

and in that the weight ratio between the sucralfate and the non steroidic antiinflammatory drug is comprised between 1:4 and 8:1.

2. Solid pharmaceutical form according to claim 1 wherein the coating layer comprises beside the sucralfate also other mucose protecting active principles selected from: mucin, cellulose derivatives, natural or synthetic polymeric materials.

3. Solid pharmaceutical form according to claim 1 wherein the non steroidic antiinflammatory drug is selected from: aspirin, Indoprofen, Naproxen, Ketoprofen, Indomethacin, Diflunisal, Diclofenac.

4. Method for the preparation of the pharmaceutical form according to one of the claims from 1 to 3, based on the double compression technique and wherein the coating layer comprising sucralfate is compressed around the previously compressed central core containing the non steroidic antiinflammatory drug.

**Claims for the Contracting State: AT**

1. Method for the preparation of solid pharmaceutical form for administration by oral way, with sequential release of the contained active principles, constituted by:

a) a central core containing as active principle a non-steroidic antiinflammatory drug which normally causes ulcerogenic side effects;

b) a coating layer for said central core, comprising as active principle sucralfate, which is immediately released in the acidic medium of the stomach,

the weight ratio between the sucralfate and the non steroidic antiinflammatory drug being comprised between 1:4 and 8:1, said method comprising the double compression technique and wherein the coating layer comprising sucralfate is compressed around the previously compressed central core containing the non steroidic antiinflammatory drug.

2. Method for the preparation of pharmaceutical form according to claim 1, characterized in that said coating layer comprises beside the sucralfate also other mucose protecting active principles selected from: mucin, cellulose derivatives, natural or synthetic polymeric materials.

3. Method for the preparation of pharmaceutical form according to claim 1, characterized in that said non steroidic antiinflammatory drug is selected from: aspirin, Indoprofen, Naproxen, Ketoprofen, Indomethacin, Diflunisal, Diclofenac.

# EP 0 167 958 B1

## Patentansprüche

1. Feste pharmazeutische Form zur Verabreichung auf oralem Weg mit aufeinanderfolgender Freisetzung der enthaltenen Wirkstoffe, dadurch gekennzeichnet, daß sie aus

a) einem mittleren Kern, der als Wirkstoff ein nicht-steroides entzündungshemmendes Arzneimittel enthält, das gewöhnlich ulcerogene Nebenwirkungen verursacht, und

b) einer Überzugsschicht für den mittleren Kern, die als Wirkstoff Sucralfat aufweist, das im sauren Medium des Magens sofort freigesetzt wird,

besteht, wobei das Gewichtsverhältnis zwischen dem Sucralfat und dem nicht-steroiden entzündungshemmenden Arzneimittel 1:4 bis 8:1 beträgt.

2. Feste pharmazeutische Form nach Anspruch 1, worin die Überzugsschicht außer Sucralfat auch andere schleimhautschützende Wirkstoffe ausgewählt aus Mucin, Zellulosederivativen und natürlichen oder synthetischen Polymermaterialien aufweist.

3. Feste pharmazeutische Form nach Anspruch 1, worin das nicht-steroide entzündungshemmende Arzneimittel ausgewählt ist aus Aspirin, Indoprofen, Naproxen, Ketoprofen, Indomethacin, Diflunisal and Diclofenac.

4. Verfahren zum Herstellen der pharmazeutischen Form nach einem der Ansprüche 1 bis 3 basierend auf der doppelten Kompressionstechnik, worin die Sucralfat enthaltende Überzugsschicht um den vorher komprimierten, das nicht-steroide entzündungshemmende Arzneimittel enthaltenden mittleren Kern komprimiert wird.

## Patentansprüche für Österreich

1. Verfahren zur Herstellung einer festen pharmzeutischen Form zur Verabreichung auf oralem Weg mit aufeinanderfolgender Freisetzung der enthaltenen Wirkstoffe, die aus

a) einem mittleren Kern, der als Wirkstoff ein nicht-steroides entzündungshemmendes Arzneimittel enthält, das gewöhnlich ulcerogene Nebenwirkungen verursacht, und

b) einer Überzugsschicht für den mittleren Kern, die als Wirkstoff Sucralfat aufweist, das im sauren Medium des Magens sofort freigesetzt wird,

besteht, wobei das Gewichtsverhältnis zwischen dem Sucralfat und dem nicht-steroiden entzündungshemmenden Arzneimittel 1:4 bis 8:1 beträgt, welches Verfahren die doppelte Kompressionstechnik umfaßt, worin die Sucralfat enthaltende Überzugsschicht um den vorher komprimierten, das nicht-steroide entzündungshemmende Arzneimittel enthaltenden mittleren Kern komprimiert wird.

2. Verfahren zur Herstellung der pharmazeutischen Form nach Anspruch 1, dadurch gekennzeichnet, daß die Überzugsschicht außer Sucralfat auch andere schleimhautschützende Wirkstoffe ausgewählt aus Mucin, Zellulosederivaten und natürlichen oder synthetischen Polymermaterialien aufweist.

3. Verfahren zur Herstellung der pharmazeutischen Form nach Anspruch 1, dadurch gekennzeichnet, daß das nicht-steroide entzündungshemmende Arzneimittel ausgewählt ist aus Aspirin, Indoprofen, Naproxen, Ketoprofen, Indomethacin, Diflunisal und Diclofenac.

## Revendications pour: FR, DE, CH

1. Produit pharmaceutique sous forme solide, pour administration par voie orale, avec libération séquentielle des principes actifs contenue, caractérisé en ce qu'il est constitué par:

a) un noyau central, contenant en tant que principe actif un médicament anti-inflammatoire non-stéroïde, qui provoque normalement des effets secondaires ulcérogènes;

b) un couche de revêtement dudit noyau central, comportant en tant que principe actif du sucralfate, qui est immédiatement libéré dans le milieu acide de l'estomac,

et en ce que le rapport pondéral entre le sucralfate et le médicament anti-inflammatoire nonstéroïdique est compris entre 1/4 et 8/1.

2. Produit pharmaceutique sous forme solide, selon la revendication 1, dans lequel la couche de revêtement comprend, outre le sucralfate, sussi d'autres principes actifs protégeant la muqueuse, choisis parmi: la mucine, des dérivés de la cellulose, des matières polymères naturelles ou synthétiques.

3. Produit pharmaceutique sous forme solide, selon la revendication 1, dans lequel le médicament anti-inflammatoire nonstéroïdique est choisi parmi: l'Aspirine, l'Indoprofen, le Naproxen, le Kétoprofène, l'Indométacine, le Diflunisal et le Diclofénac.

4. Procédé de préparation du produit pharmaceutique selon l'une des revendications 1 à 3, basé sur la technique de double compression, et dans lequel la couche de revêtement comprenant la sucralfate est comprimée autour du noyau central précédemment comprimé, contenant le médicament anti-inflammatoire non-stéroïdien.

## Revendications pour: AT

1. Procédé de préparation de produit pharmaceutique sous forme solide, pour administration par voie orale, avec libération séquentielle des principes actifs contenus, constitué par:

a) un noyau central, contenant en tant que principe actif un médicament anti-inflammatoire non-stéroïdien, qui provoque normalement des effets secondaires ulcérogènes;

b) une couche de revêtement dudit noyau central, comportant en tant que principe actif du sucralfate, qui est immédiatement libéré dans le milieu acide de l'estomac,

le rapport pondéral entre le sucralfate et le médicament anti-inflammatoire non-stéroïdien étant compris entre 1/4 et 8/1, ledit procédé comprenant la technique de double compression et dans lequel la couche de revêtement comportant le sucralfate est comprimée autour du noyau central précédemment comprimé, contenant le médicament anti-inflammatoire non-stéroïdien.

2. Procédé de préparation de produit pharmaceutique, selon la revendication 1, caractérisé en ce que ladite couche de revêtement comprend, outre le sucralfate, aussi d'autres principes actifs protégeant la muqueuse, choisis parmi: la mucine, des dérivés de la cellulose, des matières polymères naturelles ou synthétiques.

3. Procédé de préparation du produit pharmaceutique, selon la revendication 1, caractérisé en ce que ledit médicament anti-inflammatoire, non-stéroïdien est choisi parmi: l'Aspirine, l'Indoprofen, le Naproxen, le Kétoprofène, l'Indométacine, le Diflunisal et le Diclofénac.

FIGURE 1